# EUROPEAN PATENT SPECIFICATION

(11) **EP 2 231 320 B1**
(45) Date of publication and mention of the grant of the patent: **22.05.2013**
(21) Application number: 08852474.9
(22) Date of filing: 19.11.2008
(51) Int. Cl.: B01J 8/18, B01J 8/26, C07C 1/20, C07C 11/02, B01J 29/80

(54) **PROCESS FOR THE PREPARATION OF AN OLEFINIC PRODUCT**
VERFAHREN ZUR HERSTELLUNG EINES OLEFINPRODUKTS
PROCÉDÉ DE PRÉPARATION DE PRODUIT OLÉFINIQUE

(30) Priority: 19.11.2007 EP 07121005; 19.11.2007 EP 07121003; 19.11.2007 EP 07121014; 19.11.2007 EP 07121008; 19.11.2007 EP 07120962; 19.11.2007 EP 07120963
(43) Date of publication of application: 29.09.2010
(73) Proprietor: Shell Internationale Research Maatschappij B.V., 2596 HR The Hague (NL)
(72) Inventor: VAN WESTRENEN, Jeroen, NL-1031 CM Amsterdam (NL); CHEWTER, Leslie Andrew, NL-1031 CM Amsterdam (NL); WINTER, Ferry, NL-1031 CM Amsterdam (NL)
(74) Representative: Matthezing, Robert Maarten
(86) International application number: PCT/EP2008/065876
(87) International publication number: WO 2009/065877

(56) References cited:
- EP-A- 0 485 145
- EP-A- 0 489 497
- WO-A-95/22516
- WO-A-03/020667

## Description

### Field of the Invention

This invention relates to catalyst particles, a process for the an olefinic product, and to a process for the preparation of an oxygenate conversion catalyst. The invention is useful for for the preparation of an olefin or olefinic product, especially lower olefins such as ethylene and/or propylene. In particular this invention relates to the conversion of an oxygenate feedstock into olefins.

### Background of the invention

Processes for the preparation of olefins from oxygenates are known in the art. Of particular interest is often the production of light olefins, in particular ethylene and/or propylene. The oxygenate feedstock can for example comprise methanol and/or dimethylether, and an interesting route includes their production from synthesis gas derived from e.g. natural gas or via coal gasification.

For example, WO2007/135052 discloses a process wherein an alcohol and/or ether containing oxygenate feedstock and an olefinic co-feed are reacted in the presence of a zeolite having one-dimensional 10-membered ring channels to prepare an olefinic reaction mixture, and wherein part of the obtained olefinic reaction mixture is recycled as olefinic co-feed. With a methanol and/or dimethylether containing feedstock, and an olefinic co-feed comprising C4 and/or C5 olefins, an olefinic product rich in light olefins can be obtained.

WO2004/05694 discloses a process for cracking C4-C8 hydrocarbons, to prepare olefins, especially propylene, wherein a catalyst with a combination of ZSM-12 with either ZSM-5 or ZSM-23 is used. No reference to oxygenates is made.

US 6 797 851 describes a process for making ethylene and propylene from an oxygenate feed. The process is conducted in two stages using two different zeolite catalysts, wherein in the first stage oxygenates are converted to a light olefin stream, and wherein in the second stage C4+ olefins produced in the first stage are converted to additional ethylene and propylene. The only zeolite that is disclosed for the first step is ZSM-5. For the second stage, zeolite ZSM-22 and ZSM-35 are disclosed in experiments. Various embodiments of reaction systems with first and second stage catalyst in separate reaction zones are discussed. Without disclosing an embodiment, it is generally mentioned that the two catalysts can be mixed.

### Summary of the invention

It is desired to provide a process and suitable catalyst to maximise production of light olefins, and in a particular aspect maximise the production of ethylene, from an oxygenate feedstock.

According to a first aspect of the present invention, there is provided catalyst particles comprising a first molecular sieve having one-dimensional 10-membered ring channels, and a second molecular sieve having more-dimensional channels. Thus, the individual catalyst particles comprise both the first molecular sieve and the second molecular sieve. Typically the molecular sieves comprise or consist of crystals.

According to a second aspect of the present invention, there is provided a process for the preparation of an olefinic product, which process comprises reacting an oxygenate feedstock in a reaction zone in the presence of oxygenate conversion catalyst particles to prepare an olefinic reaction product; wherein individual oxygenate conversion catalyst particles comprise both a first molecular sieve having one-dimensional 10-membered ring channels, and a second molecular sieve having more-dimensional channels, according to the first aspect of the invention..

Thus the first and second molecular sieves are intimately mixed, that is crystals of the first and second molecular sieves are present in the same particle, as opposed to a mixture of catalyst particles where individual particles include one or other of the various molecular sieve types, not both.

Preferably therefore an average distance between a crystal of the first molecular sieve and a crystal of the second molecular sieve is less than an average particle size of the catalyst particles, preferably 40 µm or less, more preferably 20 µm or less, especially 10 µm or less. For near-spherical particles the average particle size can be determined by the weight-averaged diameter of a statistically representative quantity of particles, such as of e.g. 10 mg, 100 mg, 250 mg, or 1 g of particles. Such a statistically representative quantity of particles is referred to herein as a bed of particles. For other shapes of catalyst particles the skilled person knows how to define a suitable average of a characteristic dimension as average particle size, preferably a weight-average is used.

Preferably a bed of the catalyst particles comprises at least 1 wt% and less than 50 wt% of the second molecular sieve, based on the total weight of first and second molecular sieves in the bed; preferably at least 5 wt% and less than 40 wt%, more preferably at least 8 wt% and less than 25 wt%. For certain embodiments the second molecular sieve may be present at less than 18wt% and indeed may be less than 15 wt% based on the total weight of molecular sieves in the catalyst composition.

According to a further aspect of the present invention there is provided a process for the preparation of an oxygenate conversion catalyst, the process comprising preparing oxygenate conversion catalyst particles comprising a first molecular sieve having one-dimensional 10-membered ring channels, and a second molecular sieve having more-dimensional channels such that the resulting individual catalyst particles comprise both the first molecular sieve and second molecular sieve.

To form a catalyst the first and second molecular sieves are typically embedded in a matrix. For the purposes of this invention 'matrix' is herein referred to as including any filler and/or binder components.

For certain embodiments, a mixture comprising the first and second molecular sieves and matrix are spray dried to form the catalyst particles. Typically a mixture comprising the first and second molecular sieves are milled, either separately but preferably together, before the matrix is added to form a slurry that is spray dried.

Alternatively the first and second molecular sieves are co-crystallised or intergrown. For such embodiments a matrix is typically added after co-crystallisation and the resulting mixture is then spray dried.

Preferably the catalyst particles prepared in accordance with the further aspect of the present invention are used in a process in accordance with the second aspect of the present invention.

The process of the invention allows maximising of olefin production, in particular ethylene and/or propylene production, more in particular a high ethylene make, from an , oxygenate feedstock comprising e.g. methanol and/or dimethylether. It has been found that an oxygenate conversion catalyst comprising or consisting of oxygenate conversion particles according to the present invention is particularly effective for this purpose. It has been found particularly advantageous to apply this catalyst for converting a reaction mixture comprising an olefinic co-feed in addition to the oxygenate, to an olefinic product comprising ethylene and/or propylene.

Examples of an oxygenate that can be used as feedstock in the present invention include alcohols, such as methanol, ethanol, isopropanol, ethylene glycol, propylene glycol; ketones, such as acetone and methylethylketone; aldehydes, such as formaldehyde, acetaldehyde and propionaldehyde; ethers, such as dimethylether, diethylether, methylethylether, tetrahydrofuran and dioxane; epoxides such as ethylene oxide and propylene oxide; and acids, such as acetic acid, propionic acid, formic acid and butyric acid. Further examples are dialkyl carbonates such as dimethyl carbonate or alkyl esters of carboxylic acids such as methyl formate. Of these examples, alcohols and ethers are preferred.

Examples of preferred oxygenates include alcohols, such as methanol, ethanol, isopropanol, ethylene glycol, propylene glycol; and dialkyl ethers, such as dimethylether, diethylether, methylethylether. Cyclic ethers such as tetrahydrofuran and dioxane, are also suitable.

The oxygenate used in the process according to the invention is preferably an oxygenate which comprises at least one oxygen-bonded alkyl group. The alkyl group preferably is a C1-C4 alkyl group, i.e. comprises 1 to 4 carbon atoms; more preferably the alkyl group comprises 1 or 2 carbon atoms and most preferably one carbon atom. The oxygenate can comprise one or more of such oxygen-bonded C1-C4 alkyl groups. Preferably, the oxygenate comprises one or two oxygen-bonded C1-C4 alkyl groups.

More preferably an oxygenate is used having at least one C1 or C2 alkyl group, still more preferably at least one C1 alkyl group.

Preferably the oxygenate is chosen from the group of alkanols and dialkyl ethers consisting of dimethylether, diethylether, methylethylether, methanol, ethanol and isopropanol, and mixtures thereof.

Most preferably the oxygenate is methanol or dimethylether, or a mixture thereof.

Preferably the oxygenate feedstock comprises at least 50 wt% of methanol and/or dimethylether, more preferably at least 80 wt%, most preferably at least 90 wt%.

The oxygenate feedstock can be obtained from a prereactor, which converts methanol at least partially into dimethylether. In this way, water may be removed by distillation and so less water is present in the process of converting oxygenate to olefins, which has advantages for the process design and lowers the severity of hydrothermal conditions the catalyst is exposed to.

The oxygenate feedstock can comprise an amount of water, preferably less than 10 wt%, more preferably less than 5 wt%, based on the total weight of oxygenate feedstock. Preferably the oxygenate feedstock contains essentially no hydrocarbons other than oxygenates, i.e. less than 5 wt%, preferably less than 1 wt%, based on the total weight of oxygenate feedstock.

In one embodiment, the oxygenate is obtained as a reaction product of synthesis gas. Synthesis gas can for example be generated from fossil fuels, such as from natural gas or oil, or from the gasification of coal. Suitable processes for this purpose are for example discussed in Industrial Organic Chemistry, Klaus Weissermehl and Hans-Jürgen Arpe, 3rd edition, Wiley, 1997, pages 13-28. This book also describes the manufacture of methanol from synthesis gas on pages 28-30.

In another embodiment the oxygenate is obtained from biomaterials, such as through fermentation. For example by a process as described in DE-A-10043644.

In a particular embodiment the oxygenate feedstock is reacted to produce the olefinic product in the presence of an olefinic co-feed. By an olefinic composition or stream, such as an olefinic product, product fraction, fraction, effluent, reaction product or the like is understood a composition or stream comprising one or more olefins, unless specifically indicated otherwise. Other species can be present as well. Apart from olefins, the olefinic co-feed may contain other hydrocarbon compounds, such as for example paraffinic compounds. Preferably the olefinic co-feed comprises an olefinic portion of more than 50 wt%, more preferably more than 60 wt%, still more preferably more than 70 wt%, which olefinic portion consists of olefin(s). The olefinic co-feed can also consist essentially of olefin(s).

Any non-olefinic compounds in the olefinic co-feed are preferably paraffinic compounds. Such paraffinic compounds are preferably present in an amount in the range from 0 to 50 wt%, more preferably in the range from 0 to 40 wt%, still more preferably in the range from 0 to 30 wt%.

By an olefin is understood an organic compound containing at least two carbon atoms connected by a double bond. The olefin can be a mono-olefin, having one double bond, or a poly-olefin, having two or more double bonds. Preferably olefins present in the olefinic co-feed are mono-olefins. C4 olefins, also referred to as butenes (1-butene, 2-butene, iso-butene, and/or butadiene), in particular C4 mono-olefins, are preferred components in the olefinic co-feed.

Preferably, when an olefinic co-feed is used, it is at least partially obtained by a recycle stream formed by recycling a suitable fraction of the reaction product comprising C4 olefin. The skilled artisan knows how to obtain such a fraction from the olefinic reaction product such as by distillation.

In one embodiment at least 70 wt% of the olefinic co-feed, during normal operation, is formed by the recycle stream, preferably at least 90 wt%, more preferably at least 99 wt%. Most preferably the olefinic co-feed is during normal operation formed by the recycle stream, so that the process converts oxygenate feedstock to predominantly light olefins without the need for an external olefins stream. During normal operation means for example in the course of a continuous operation of the process, for at least 70% of the time on stream. The olefinic co-feed may need to be obtained from an external source, such as from a catalytic cracking unit or from a naphtha cracker, during start-up of the process, when the reaction product comprises no or insufficient C4+ olefins.

The C4 fraction contains C4 olefin(s), but can also contain a significant amount of other C4 hydrocarbon species, in particular C4 paraffins, because it is difficult to economically separate C4 olefins and paraffins, such as by distillation.

In a preferred embodiment the olefinic co-feed and preferably also the recycle stream comprises C4 olefins and less than 10 wt% of C5+ hydrocarbon species, more preferably at least 50 wt% of C4 olefins, and at least a total of 70 wt% of C4 hydrocarbon species.

The olefinic co-feed and preferably also the recycle stream, can in particular contain at least a total of 90 wt% of C4 hydrocarbon species. In a preferred embodiment, the olefinic co-feed comprises less than 5 wt% of C5+ olefins, preferably less than 2 wt% of C5+ olefins, even more preferably less than 1 wt% of C5+ olefins, and likewise the recycle stream. In another preferred embodiment, the olefinic co-feed, comprises less than 5 wt% of C5+ hydrocarbon species, preferably less than 2 wt% of C5+ hydrocarbon species even more preferably less than 1 wt% of C5+ hydrocarbon species, and likewise the recycle stream.

Thus in certain preferred embodiments, the olefinic portion of the olefinic co-feed, and of the recycle stream, comprises at least 90 wt% of C4 olefins, more preferably at least 99 wt%. Butenes as co-feed have been found to be particularly beneficial for high ethylene selectivity. Therefore one particularly suitable recycle stream consists essentially, i.e. for at least 99 wt%, of 1-butene, 2-butene (cis and trans), isobutene, n-butane, isobutene, butadiene.

In certain embodiments, the recycle stream can also comprise propylene. This may be preferred when a particularly high production of ethylene is desired, so that part or all of the propylene produced, such as at least 5 wt% thereof, is recycled together with C4 olefins.

The preferred molar ratio of oxygenate in the oxygenate feedstock to olefin in the olefinic co-feed depends on the specific oxygenate used and the number of reactive oxygen-bonded alkyl groups therein. Preferably the molar ratio of oxygenate to olefin in the total feed lies in the range of 10:1 to 1:10, more preferably in the range of 5:1 to 1:5 and still more preferably in the range of 3:1 to 1:3.

In a preferred embodiment wherein the oxygenate comprises only one oxygen-bonded methyl group, such as methanol, the molar ratio preferably lies in the range from 5:1 to 1:5 and more preferably in the range of 2.5:1 to 1:2.5.

In another preferred embodiment wherein the oxygenate comprises two oxygen-bonded methyl groups, such as for example dimethylether, the molar ratio preferably lies in the range from 5:2 to 1:10 and more preferably in the range of 2:1 to 1:4. Most preferably the molar ratio in such a case is in the range of 1.5:1 to 1:3.

The expression 'molecular sieve' is used in the description and claims for a material containing small regular pores and/or channels and exhibiting catalytic activity in the conversion of oxygenate to olefin. The first molecular sieve having one-dimensional 10-membered ring channels and/or the second molecular sieve having more-dimensional channels ("more-dimensional molecular sieve") can in particular be a zeolite or zeolites. A zeolite is understood to be an aluminosilicate molecular sieve. Where reference is made in the description and in the claims to a molecular sieve, this can in particular be a zeolite. The first molecular sieve having one-dimensional 10-membered ring channels and/or the second molecular sieve having more-dimensional channels can be a mixture of different types of molecular sieves having the respective channel structures. So, for example, a mixture of ZSM-22 and ZSM-23 zeolites, both having one-dimensional 10-membered ring channels, can be used as first molecular sieve. Similarly, different more-dimensional molecular sieves can be mixed to form the second molecular sieve.

The process to prepare an olefin is carried out in presence of the first molecular sieve having one-dimensional 10-membered ring channels. These are understood to be molecular sieves having only 10-membered ring channels in one direction, which are not intersected by other channels, in particular other 8, 10 or 12-membered ring channels, from another direction.

Preferably, the first molecular sieve is a zeolite especially one selected from the group of TON-type (for example zeolite ZSM-22), MTT-type (for example zeolite ZSM-23), STF-type (for example SSZ-35), SFF-type (for example SSZ-44), EUO-type (for example ZSM-50), and EU-2-type molecular sieves or mixtures thereof.

MTT-type catalysts are more particularly described in e.g. US-A-4,076,842. For purposes of the present invention, MTT is considered to include its isotypes, e.g., ZSM-23, EU-13, ISI-4 and KZ-1.

TON-type molecular sieves are more particularly described in e.g. US-A-4,556,477. For purposes of the present invention, TON is considered to include its isotypes, e.g., ZSM-22, Theta-1, ISI-1, KZ-2 and NU-10.

EU-2-type molecular sieves are more particularly described in e.g. US-A-4,397,827. For purposes of the present invention, EU-2 is considered to include its isotypes, e.g., ZSM-48.

In a further preferred embodiment a first molecular sieve of the MTT-type, such as ZSM-23, and/or a TON-type, such as ZSM-22 is used.

Molecular sieve and zeolite types are for example defined in Ch. Baerlocher and L.B. McCusker, Database of Zeolite Structures: http://www.iza-structure.org/databases/, which database was designed and implemented on behalf of the Structure Commission of the International Zeolite Association (IZA-SC), and based on the data of the 4th edition of the Atlas of Zeolite Structure Types (W.M. Meier, D.H. Olson and Ch. Baerlocher).

The second molecular sieve having more-dimensional channels is understood to have intersecting channels in at least two directions. So, for example, the channel structure is formed of substantially parallel channels in a first direction, and substantially parallel channels in a second direction, wherein channels in the first and second directions intersect. Intersections with a further channel type are also possible. Preferably the channels in at least one of the directions are 10-membered ring channels. The second molecular sieve can be for example a FER type zeolite which is a two-dimensional structure and has 8- and 10-membered rings intersecting each other. Preferably however the intersecting channels in the second molecular sieve are each 10-membered ring channels. Thus the second molecular sieve may be a zeolite, or a SAPO-type (silicoaluminophosphate) molecular sieve. More preferably however the second molecular sieve is a zeolite. A preferred second molecular sieve is an MFI-type zeolite, in particular zeolite ZSM-5.

The presence of the second molecular sieve in the oxygenate conversion catalyst particles was found to improve stability (slower deactivation during extended runs) and hydrothermal stability compared to a catalyst with only the one-dimensional molecular sieve and without the more-dimensional molecular sieve. Without wishing to be bound by a particular hypothesis or theory, it is presently believed that this is due to the possibility for converting larger molecules by the second molecular sieve having more-dimensional channels, that were produced by the first molecular sieve having one-dimensional 10-membered ring channels, and which would otherwise form coke. Moreover the intimate mix of the first with the second molecular sieve, such that both are present in the individual catalyst particles, was found to improve the selectivity towards ethylene and propylene, more in particular towards ethylene.

The weight ratio between the first molecular sieve having one-dimensional 10-membered ring channels, and the second molecular sieve having more-dimensional channels can be in the range of from 1:100 to 100:1. Preferably, the first molecular sieve is present in a weight majority. The weight ratio of first to second molecular sieve can be of from 1:1 to 100:1, more preferably in the range of from 9:1 to 2:1.

In preferred embodiments the second molecular sieve is an MFI-type molecular sieve, in particular zeolite ZSM-5, having a Silica-to-Alumina ratio SAR of at least 60, more preferably at least 80, even more preferably at least 100, yet more preferably at least 150. At higher SAR the percentage of C4 saturates in the C4 totals produced is minimized. In special embodiments the oxygenate conversion catalyst particles can comprise less than 35 wt% of the second molecular sieve, based on the total molecular sieve in the oxygenate conversion catalyst, in particular less than 20 wt%, more in particular less than 18 wt%, still more in particular less than 15 wt%. Suitably at least 1 wt% of the second molecular sieve, based on the total molecular sieve, is present in the particles.

In one embodiment the oxygenate conversion catalyst can comprise more than 50 wt%, preferably at least 65 wt%, based on total molecular sieve in the oxygenate conversion catalyst, of the molecular sieve having one-dimensional 10-membered ring channels. The presence of a majority of such molecular sieve strongly determines the predominant reaction pathway.

Without wishing to be bound by a particular hypothesis or theory, it is currently believed that the reaction is dominated by a majority portion of the molecular sieve having one-dimensional 10-membered ring channels. In such molecular sieve an alcohol or ether oxygenate can be converted to an olefinic product by an initial alkylation step with an olefin from the olefinic co-feed, followed by cracking. The presence of a minority portion of a more-dimensional molecular sieve in the oxygenate conversion catalyst particles was found sufficient to significantly improve stability and hydrothermal stability compared to a catalyst with only the one-dimensional molecular sieve and without the more-dimensional molecular sieve.

In one embodiment, the first and second molecular sieves are used in their hydrogen form in the oxygenate conversion catalyst, e.g., HZSM-22, HZSM-23, and HZSM-48, HZSM-5. Preferably at least 50% w/w, more preferably at least 90% w/w, still more preferably at least 95% w/w and most preferably 100% of the total amount of molecular sieve used is in the hydrogen form. When the molecular sieves are prepared in the presence of organic cations the molecular sieve may be activated by heating in an inert or oxidative atmosphere to remove organic cations, for example, by heating at a temperature over 500 °C for 1 hour or more. The sieves are typically obtained in the sodium or potassium form. The hydrogen form can then be obtained by an ion exchange procedure with ammonium salts followed by another heat treatment, for example in an inert or oxidative atmosphere at a temperature over 300°C. The molecular sieves obtained after ion-exchange are also referred to as being in the ammonium form.

In a preferred embodiment the first molecular sieve having one-dimensional 10-membered ring channels comprises at least one of a molecular sieve of the MTT-type and/or of the TON-type. Examples are ZSM-23 for MTT, and ZSM-22 for TON.

Suitably the molecular sieve having one-dimensional 10-membered ring channels has a Silica-to-Alumina ratio (SAR) in the range from 1 to 500. A particularly suitable SAR is less than 200, in particular 150 or less. A preferred range is from 10 to 200 or from 10-150. The SAR is defined as the molar ratio of SiO₂/Al₂O₃ corresponding to the composition of the molecular sieve.

For ZSM-22, a SAR in the range of 40-150 is preferred, in particular in the range of 70-120. Good performance in terms of activity and selectivity has been observed with a SAR of about 100.

For ZSM-23, a SAR in the range of 20-120 is preferred, in particular in the range of 30-80. Good performance in terms of activity and selectivity has been observed with a SAR of about 50.

Preferably the second molecular sieve having more-dimensional channels has a silica-to-alumina ratio (SAR) in the range from 1 to 1000. For ZSM-5, a SAR of 60 or higher is preferred, in particular 80 or higher, more preferably 100 or higher, still more preferably 150 or higher, such as 200 or higher.

The catalyst particles are typically formulated from the molecular sieves, such as in a mixture or in combination within a matrix component such as a so-called binder material and/or a filler material. Other components can also be present in the formulation.

It is desirable to provide a catalyst having good mechanical or crush strength, because in an industrial environment the catalyst is often subjected to rough handling, which tends to break down the catalyst into powder-like material. The latter causes problems in the processing. Preferably the molecular sieve is therefore incorporated within a matrix such as a binder material. Examples of suitable materials in a formulation include active and inactive materials and synthetic or naturally occurring zeolites as well as inorganic materials such as clays, silica, alumina, silica-alumina, titania, zirconia and aluminosilicate or mixtures thereof. For present purposes, inert materials, such as silica, are preferred because they may prevent unwanted side reactions which may take place in case a more acidic material, such as alumina or silica-alumina is used.

Preferably the matrix and therefore the formulated catalyst particles (i.e. the combination of the first and second molecular sieves and the matrix) comprise non-zeolitic components. Typically at least 5wt% of the formulated catalyst comprises non-zeolitic components, preferably at least 20wt%.

Preferably the total molecular sieve content, especially where the molecular sieves are both zeolites, is at most 60wt% of the formulated catalyst particles, especially at most 50wt%; typically at least 10wt%.

Silica binder is especially preferred where the molecular sieve comprises ZSM-22.

Now the aspect of the present invention relating to the process for the preparation of an olefinic product will be discussed in more detail, which process comprises reacting an oxygenate feedstock in a reaction zone in the presence of catalyst particles according to the invention.

The process for the preparation of an olefinic product of the present invention can be carried out in a batch, continuous, semi-batch or semi-continuous manner. Preferably the process of the present invention is carried out in a continuous manner.

Since a second molecular sieve having more-dimensional channels such as ZSM-5 is present in the oxygenate conversion catalyst particles, start up is possible without an olefinic co-feed from an external source. ZSM-5 for example is able to convert an oxygenate to an olefin-containing product, so that a recycle can be established. Molecular sieves with one-dimensional 10-membered ring channels such as ZSM-22 or ZSM-23 are typically not able to convert an oxygenate feed to an olefinic product stream with any useful conversion, unless an olefinic co-feed is provided.

In a particular embodiment an olefinic co-feed obtained from an external source may be used at start-up, and/or after start-up. Such olefins may for example be obtained from a steam cracker, a catalytic cracker, alkane dehydrogenation (e.g. propane or butane dehydrogenation). Further, such olefins can be bought from the market. In a special embodiment the olefins for such start-up are obtained from a previous process that converted oxygenates, with or without olefinic co-feed, to olefins. Such a previous process may have been located at a different location or it may have been carried out at an earlier point in time.

Typically the oxygenate conversion catalyst deactivates in the course of the process. Conventional catalyst regeneration techniques can be employed. The catalyst particles used in the process of the present invention can have any shape known to the skilled person to be suitable for this purpose, for it can be present in the form of spray dried catalyst particles, spheres, tablets, rings, extrudates, etc. Extruded catalysts can be applied in various shapes, such as, cylinders and trilobes. If desired, spent oxygenate conversion catalyst can be regenerated and recycled to the process of the invention.

Spherical particles are normally obtained by spray drying. Preferably the average particle size is in the range of 1 - 200 µm, preferably 50 - 100 µm.

The reaction zone for the oxugenate conversion reaction is typically in a reactor system, which may comprise any suitable reactor known to the skilled person and may for example contain a fixed bed, moving bed, fluidized bed, riser reactor and the like. A riser reactor system is preferred, in particular a riser reactor system comprising a plurality of serially arranged riser reactor stages.

In processes where a riser reactor system is preferred, a catalyst is required which has high attrition resistance to limit the catalyst losses by attrition. Such catalyst is typically formed of spraydried catalyst particles. The composition of the catalyst particles strongly influence their resistance to attrition.

The reaction to convert oxygenates and optionally the olefinic co-feed to an olefinic product can be carried out over a wide range of temperatures and pressures. Suitably, however, the oxygenate feed and optional olefinic co-feed are contacted with the molecular sieve at a temperature in the range from 200 °C to 650 °C. In a further preferred embodiment the temperature is in the range from 250°C to 600 °C, more preferably in the range from 300°C to 550 °C, most preferably in the range from 450°C to 550 °C. Preferably the reaction to produce the olefins is conducted at a temperature of more than 450°C, preferably at a temperature of 460°C or higher, more preferably at a temperature of 490°C or higher. At higher temperatures a higher activity and ethylene selectivity is observed. Molecular sieves having one-dimensional 10-membered ring channels can be operated under oxygenate conversion conditions at such high temperatures with acceptable deactivation due to coking, contrary to molecular sieves with smaller pores or channels, such as 8-membered ring channels. Temperatures referred to hereinabove represent reaction temperatures, and it will be understood that a reaction temperature can be an average of temperatures of various feed streams and the catalyst in the reaction zone.

In addition to the oxygenate, and the olefinic co-feed, a diluent may be fed into the reactor system. It is preferred to operate without a diluent, or with a minimum amount of diluent, such as less than 200 wt% of diluent based on the total amount of oxygenate feed, in particular less than 100 wt%, more in particular less than 20 wt%. Any diluent known by the skilled person to be suitable for such purpose can be used. Such diluent can for example be a paraffinic compound or mixture of compounds. Preferably, however, the diluent is an inert gas. The diluent can be argon, nitrogen, and/or steam. Of these, steam is the most preferred diluent. For example, the oxygenate feed and optionally olefinic co-feed can be diluted with steam, for example in the range from 0.01 to 10 kg steam per kg oxygenate feed.

In one embodiment small amounts of water are added in order to improve the stability of the catalyst by reducing coke formation.

The olefinic reaction product is typically fractionated. The skilled artisan knows how to separate a mixture of hydrocarbons into various fractions, and how to work up fractions further for desired properties and composition for further use. The separations can be carried out by any method known to the skilled person in the art to be suitable for this purpose, for example by vapour-liquid separation (e.g. flashing), distillation, extraction, membrane separation or a combination of such methods. Preferably the separations are carried out by means of distillation. It is within the skill of the artisan to determine the correct conditions in a fractionation column to arrive at such a separation. He may choose the correct conditions based on, inter alia, fractionation temperature, pressure, trays, reflux and reboiler ratios.

At least a light olefinic fraction comprising ethylene and/or propylene and a heavier olefinic fraction comprising C4 olefins are normally obtained. The heavier olefinic fraction preferably contains less than 10 wt% of C5+ hydrocarbon species. Preferably also a water-rich fraction is obtained. Also a lighter fraction comprising methane, carbon monoxide, and/or carbon dioxide can be obtained, as well as one or more heavy fractions comprising C5+ hydrocarbons. Such heavy fraction can for example be used as gasoline blending component.

In a particular aspect the present invention provides a process for the preparation of an olefinic product, which process comprises the step a) of reacting an oxygenate feedstock and an olefinic co-feed in a reactor in the presence of oxygenate conversion catalyst particles comprising both a first molecular sieve having one-dimensional 10-membered ring channels, and a second molecular sieve having more-dimensional channels, to prepare an olefinic reaction effluent. Preferably the weight ratio between the one-dimensional molecular sieve and the further molecular sieve is in the range of from 1:1 to 100:1. In a preferred embodiment, this process comprises the further steps of b) separating the olefinic reaction effluent into at least a first olefinic fraction and a second olefinic fraction; and c) recycling at least part of the second olefinic fraction obtained in step b) to step a) as olefinic co-feed; and d) recovering at least part of the first olefinic fraction obtained in step b) as olefinic product.

In step b) of this process according to the invention the olefinic reaction effluent of step a) is separated (fractionated). At least a first olefinic fraction and a second olefinic fraction, preferably containing C₄ olefins, are obtained. The first olefinic fraction typically is a light olefinic fraction comprising ethylene, and the second olefinic fraction is typically a heavier olefinic fraction comprising C4 olefins.

Preferably also a water-rich fraction is obtained. Also a lighter fraction comprising contaminants such as methane, carbon monoxide, and/or carbon dioxide can be obtained and withdrawn from the process, as well as one or more heavy fractions comprising C5+ hydrocarbons, including C5+ olefins. Such heavy fraction can for example be used as gasoline blending component. For example, the first olefinic fraction can comprise at least 50 wt%, preferably at least 80 wt%, of C1-C3 species, the recycled part of the second olefinic fraction can comprise at least 50 wt% of C₄ species, a heavier carbonaceous fraction that is withdrawn from the process can comprise at least 50 wt% of C₅₊ species.

In step c) at least part of the second olefinic fraction, preferably containing C₄ olefins, obtained in step b) is recycled to step a) as olefinic co-feed.

Only part of the second olefinic fraction or the complete second olefinic fraction may be recycled to step a).

In the process also a significant amount of propylene is normally produced. The propylene can form part of the light olefinic fraction comprising ethene, and which can suitably be further fractionated into various product components. Propylene can also form part of the heavier olefinic fraction comprising C4 olefins.

The various fractions and streams referred to herein, in particular the recycle stream, can be obtained by fractionating in various stages, and also by blending streams obtained during the fractionation. Typically, an ethylene- and a propylene-rich stream of predetermined purity such as export quality will be obtained from the process, e.g. from a C2 or C3 splitter, and also a stream rich in C4 comprising C4 olefins and optionally C4 paraffins, such as an overhead stream from a debutaniser column receiving the bottom stream from a depropanizer column at their inlet. It shall be clear that the heavier olefinic fraction comprising C4 olefins, forming the recycle stream, can be composed from quantities of various fractionation streams. So, for example, some amount of a propylene-rich stream can be blended into a C4 olefin-rich stream. In a particular embodiment at least 90 wt% of the heavier olefinic fraction comprising C4 olefins can be the formed by the overhead stream from a debutaniser column receiving the bottom stream from a depropanizer column at their inlet, more in particular at least 99 wt% or substantially all.

Suitably the olefinic reaction product comprises less than 10 wt%, preferably less than 5 wt%, more preferably less than 1 wt%, of C6-C8 aromatics. Producing low amounts of aromatics is desired since any production of aromatics consumes oxygenate which is therefore not converted to lower olefins.

The present invention will now be discussed in more detail and by way of example at the hand of several embodiments.

In these examples dimethyl ether (DME) and 1-butene were reacted over oxygenate conversion catalysts comprising particles, the catalysts containing 40 wt% of total zeolite (molecular sieve) and a matrix of 36 wt% kaolin clay (as filler) and 24 wt% of silica binder. The catalysts were prepared by spray drying wherein the weight-based average particle size is between 70-90 µm.

### Example 1:

This catalyst with particles in accordance with the invention was spray dried, wherein the zeolite component was obtained by mixing MTT (ZSM-23 SAR 46) and the MFI (ZSM-5 SAR 280) zeolites, so that the final catalyst was intimately mixed, meaning that crystals of both zeolites are present in individual catalyst particles. The MTT /MFI weight ratio is 80/20, based on total zeolite present in the catalyst.

### Example 2:

This catalyst in accordance with the invention was prepared as detailed in example 1 but with an MTT / MFI weight ratio of 62.5/37.5 based on total zeolite present in the catalyst.

### Comparative Example 3

This catalyst comprises 40 wt% of MTT zeolite (ZSM-23 SAR 46) and no other molecular sieves, and catalyst particles were otherwise prepared by spray drying as in Example 1.

### Comparative Example 4

The catalyst comprises 40 wt% of MFI zeolite (ZSM-5 SAR 280) and no other molecular sieves, and catalyst particles were otherwise prepared by spray drying as in Example 1.

### Comparative Example 5

This catalyst consists of a physical mixture of catalyst particles of the two catalysts of examples 3 and 4 above which were spray dried separately and then mixed to produce a physical mixture of catalyst particles. Thus, for physical mixtures each particle consists of either the first or the second zeolite, not both. In contrast, the intimately mixed examples 1 and 2 in accordance with the present invention have particles comprising both the first and second zeolites. The catalyst powders catalyst 3/catalyst 4 were mixed in an 80/20 weight ratio.

### Comparative Example 6

This catalyst was prepared as detailed in example 5 (i.e. a physical mixture of the catalysts in examples 3 and 4) but mixed with a 62.5/37.5 weight ratio of catalyst 3/catalyst 4.

For performance evaluation a sample of the respective catalyst powder was pressed into tablets and the tablets were broken into pieces and sieved.

The various catalysts were tested to determine the selectivity towards ethylene and propylene from oxygenates and stability under such reaction conditions. For the catalytic testing, the sieve fraction of 40-60 mesh was used. Prior to reaction, the fresh catalyst in its ammonium-form was treated ex-situ in air at 600 °C for 2 hours.

The reaction was performed using a quartz reactor tube of 3.6 mm internal diameter. The catalyst was heated in argon to 525 °C and a mixture consisting of 3 vol% dimethyl ether, 3 vol% 1-butene, 1 vol% steam balanced in N₂ was passed over the catalyst at atmospheric pressure (1 bar). The Gas Hourly Space Velocity (GHSV) is determined by the total gas flow over the catalyst weight per unit time (ml.g_{catalyst}⁻¹.h⁻¹). The effluent from the reactor was analyzed by gas chromatography (GC) to determine the product composition. The composition has been calculated on a weight basis of all hydrocarbons analyzed. The selectivity has been defined by the division of the mass of product by the sum of the masses of all products.

Results from the catalytic testing are shown in table 1.

**Table 1**

| | Catalyst 1 | | | Catalyst 2 | Catalyst 3 (Comparative) | | | Catalyst 4 (Comparative) | | | Catalyst 5 (Comparative) | | | Catalyst 6 (Comparative) |
|---|---|---|---|---|---|---|---|---|---|---|---|---|---|---|
| Time-on-stream (h) | ~0.5 | ~10 | ~ 40 | ~0.5 | ~0.5 | ~10 | ~40 | ~0.5 | ~10 | ~40 | ~0.5 | ~10 | ~40 | ~0.5 |
| DME conversion (%) | 100 | 100 | 100 | 100 | 100 | 99.5 | 37.2 | 100 | 100 | 100 | 100 | 100 | 100 | 100 |
| C2⁼/C3⁼ wt-ratio | 0. 40 | 0.28 | 0.19 | 0.34 | 0.36 | 0.15 | 0.37 | 0.30 | 0.27 | 0.24 | 0.33 | 0.23 | 0.15 | 0.29 |
| Methane (wt%) | 0.7 | 0.5 | 0.5 | 0.6 | 1.1 | 1.6 | 0.5 | 0.5 | 0.4 | 0.5 | 0.8 | 0.4 | 0.5 | 0.5 |
| C2⁼ + C3⁼ totals wt% ⁽ⁱ⁾ | 64.0 | 58.4 | 47.4 | 61.5 | 59.2 | 28.8 | 0.5 | 62.5 | 61.4 | 58.0 | 55.5 | 51.1 | 41.9 | 57.2 |
| C2 - C5 totals wt% ⁽ⁱ⁾ | 94.3 | 94.1 | 92.2 | 93.7 | 93.8 | 85.8 | 55 | 93.6 | 94.5 | 93.9 | 90.9 | 94.6 | 92.4 | 92.5 |
| wt% C4ₛₐₜₛ of C4ₜₒₜ | 6.3 | 5.9 | 7.3 | 8.2 | 4.7 | 3.4 | n.a. | 12.4 | 10.9 | 10.5 | 13.3 | 6.4 | 7.5 | 11.0 |
| C6+totals ⁽ⁱ⁾ | 5.0 | 5.4 | 7.3 | 5.6 | 5.1 | 12.3 | 2.9 | 6.1 | 5.1 | 5.6 | 8.3 | 5.0 | 7.1 | 7.0 |
| | | | | | | | | | | | | | | |
| GHSV (ml/g/h)⁽ⁱⁱ⁾ | 6, 000 | | | 6, 000 | 6, 000 | | | 6, 000 | | | 6, 000 | | | 6, 000 |
| Catalyst intake (mg) | 250 | | | 250 | 250 | | | 250 | | | 250 | | | 250 |
| DME breakthrough (h) | >58 h on stream | | | stream > 58 h on stream | 13 h on stream | | | > 58 h on stream | | | > 58 h on stream | | | 36 h on stream |

| | | | | | | | | | | | | | | |
|---|---|---|---|---|---|---|---|---|---|---|---|---|---|---|
| ⁽ⁱ⁾ wt% in product stream on carbon basis ⁽ⁱⁱ⁾ Gas hourly space velocity is based on total flow per weight of catalyst per hour (ml.g⁻¹.h⁻¹). n.a.= not accurate | | | | | | | | | | | | | | |

In Table 1 Cn refers to hydrocarbon species having n carbon atoms, Cn+ refers to hydrocarbon species having n or more carbon atoms (n being an integer) figures include all; Cn= refers to olefinic hydrocarbon species having n carbon atoms. The index sats refers to saturated carbon species, and tot or totals refer to all respective hydrocarbon species.

Thus it can be observed from table 1 that the intimately mixed catalysts in accordance with the present invention, i.e. catalysts 1 and 2, produce better results than the comparative catalysts 3 - 6. A particular advantage is that the amount of ethylene and propylene after 0.5 hours is highest for catalyst 1. At the same time the ethylene/propylene ratio C2=/C3= is highest for catalysts 1 and 2. Performance at short times on stream is very relevant for continuous processes such as fluidized bed with continuous regeneration of part or all of the catalyst.

Moreover, the stability with respect to catalyst 3 is much improved by the addition of more-dimensional zeolite to the catalyst, as can be seen from the increase of the DME breakthrough time well above the 13h for catalyst 3.

With respect to catalyst 4, the catalyst according to the present invention provides a significantly higher amount of ethylene in the product, also a higher ethylene/propylene ratio, in particular at short time on stream. Moreover, the MFI based catalyst 4 produces a much higher amount of saturates, in particular C4 saturates. A high amount of saturates is a particular disadvantages, not only as it lowers the overall conversion to olefins, but also when (part of) the C4 fraction of the product is to be recycled as olefinic co-feed. C4 saturates would build up in the reaction system much more.

Further experiments have shown that catalyst 4 also produces more aromatics. In the experiments using a diluted feed presented in Table 1, this effect is not very pronounced; aromatics contribute to the C6+ fraction in Table 1. However, it was found that in fluid bed experiments using catalyst 4 and a non-diluted feed of DME/butene (1:1 mol/mol at 525 C and a weight hourly space velocity of 8 g_{feed}.g_{cat}⁻¹.h⁻¹, 17 wt% of aromatics were formed, whereas catalyst 1 only produced 9.8 wt% of aromatics under the same conditions.

The strength of the catalyst particles is measured by their attrition resistance using a test procedure described by Weeks et al. in Oil & Gas Journal 88 (16) 1990. Catalysts 1 - 4 were first subjected to a calcination at 600 °C for 2 hours. Each test was run with 6 grams of humidified catalyst. The samples were subjected to abrasion for 36 minutes by putting the catalyst in a jet-cup using high velocity air. In this process, wear on the particles occurs as the particles collide with the jet-cup wall and each other with high velocity. The fines formed (less than 16 micrometer) are removed from the cup and collected. The formation of fines is determined over time at 5, 12, 24 and 36 minutes. An attrition index of the sample was determined by using the slope of the accumulated catalyst fines recorded at the given times. An index of less than 20 is indicated as a catalyst with good resistance for attrition. The attrition rate indicated in Table 1 is determined by taking the fines formed after 24 minutes minus the fines formed after 5 minutes divided by the total catalyst sample and divided by 0.317 hour. The attrition index and the attrition rate for catalyst 1-4 are given in Table 2.

**Table 2. Results from attrition tests**

| Catalyst | 1 | 2 | 3 (Comparative) | 4 (Comparative) |
|---|---|---|---|---|
| Attrition index | 17 | 17 | 13 | 29 |
| Attrition rate (wt%/h) | 16.1 | 15.9 | 11.2 | 26.9 |

From table 2 it can be observed that all catalysts containing the MTT zeolite with or without the MFI zeolite have good attrition resistance. Catalyst 4 having only the MFI zeolite shows poor attrition resistance. It is therefore desirable to have at least the MTT zeolite present to obtain a catalyst with sufficient attrition resistance to be applied in a riser reactor.

## Claims

1. A process for the preparation of an olefinic product, which process comprises reacting an oxygenate feedstock in a reaction zone in the presence of catalyst particles, wherein the individual catalyst particles comprise both a first molecular sieve having one-dimensional 10-membered ring channels and a second molecular sieve having more-dimensional channels, to prepare an olefinic reaction product.

2. A process according to claim 1, wherein the first molecular sieve has a Silica-to-Alumina ratio of less than 200, preferably 150 or less.

3. A process according to any one of the preceding claims, wherein an average distance between a crystal of the first molecular sieve and a nearest crystal of the second molecular sieve is less than an average particle size of the catalyst particles, preferably 40 µm or less, preferably 20 µm or less, especially 10 µm or less.

4. A process according to any one of the preceding claims, wherein the molecular sieve having one-dimensional 10-membered ring channels comprises at least one of a molecular sieve of the MTT-type and the TON-type, in particular zeolite ZSM-23 and/or zeolite ZSM-22.

5. A process according to any one of the preceding claims, wherein the second molecular sieve is an MFI-type molecular sieve, in particular zeolite ZSM-5.

6. A process according to claim 5, wherein the second molecular sieve has a Silica-to-Alumina ratio (SAR) of at least 60, preferably at least 80, more preferably at least 100, even more preferably at least 150.

7. A process according to any one of the preceding claims, wherein a bed of catalyst particles comprises at least 1 wt% and less than 50 wt% of the second molecular sieve, based on the total weight of
first and second molecular sieves in the catalyst composition; preferably at least 5 wt% and less than 40 wt%, more preferably at least 8 wt% and less than 25 wt%.

8. A process according to any one of the preceding claims, wherein the catalyst particles further comprise a matrix and a total of 60 wt% or less of the first and second molecular sieves, based on the total catalyst particles, preferably in the range of from 15 to 50wt%.

9. Process according to any one of the preceding claims, wherein the reaction of the oxygenate feedstock is performed in the presence of an olefinic co-feed.

## Patentansprüche

1. Verfahren zur Herstellung eines Olefinprodukts, welches Verfahren das Umsetzen eines Oxygenat-Einsatzmaterials in einer Reaktionszone in Gegenwart von Katalysatorteilchen umfasst, wobei die einzelnen Katalysatorteilchen sowohl ein erstes Molekularsieb mit eindimensionalen 10-gliedrigen Ring-Kanälen als auch ein zweites Molekularsieb mit mehrdimensionalen Kanälen umfassen, um ein Olefin-Reaktionsprodukt herzustellen.

2. Verfahren nach Anspruch 1, wobei das erste Molekularsieb ein Siliziumoxid-zu-Aluminiumoxid-Verhältnis von weniger als 200, vorzugsweise von 150 oder weniger, aufweist.

3. Verfahren nach einem der vorstehenden Ansprüche, wobei eine durchschnittliche Entfernung zwischen einem Kristall des ersten Molekularsiebs und einem nächstliegenden Kristall des zweiten Molekularsiebs geringer als die durchschnittliche Teilchengröße der Katalysatorteilchen, vorzugsweise 40 µm oder weniger, vorzugsweise 20 µm oder weniger, insbesondere 10 µm oder weniger, ist.

4. Verfahren nach einem der vorstehenden Ansprüche, wobei das Molekularsieb mit eindimensionalen 10-gliedrigen Ring-Kanälen wenigstens ein Molekularsieb des MTT-Typs und des TON-Typ, insbesondere Zeolith ZSM-23 und/oder Zeolith ZSM-22, umfasst.

5. Verfahren nach einem der vorstehenden Ansprüche, wobei das zweite Molekularsieb ein Molekularsieb vom MFI-Typ, insbesondere Zeolith ZSM-5, ist.

6. Verfahren nach Anspruch 5, wobei das zweite Molekularsieb ein Siliziumoxid-zu-Aluminiumoxid-Verhältnis (SAR) von wenigstens 60, vorzugsweise von wenigstens 80, stärker bevorzugt von wenigstens 100, noch stärker bevorzugt von wenigstens 150, aufweist.

7. Verfahren nach einem der vorstehenden Ansprüche, wobei ein Bett von Katalysatorteilchen wenigstens 1 Gew.-% und weniger als 50 Gew.-% des zweiten Molekularsiebs, bezogen auf das Gesamtgewicht an ersten und zweiten Molekularsieben in der Katalysatorzusammensetzung, vorzugsweise wenigstens 5 Gew.-% und weniger als 40 Gew.-%, stärker bevorzugt wenigstens 8 Gew.-% und weniger als 25 Gew.-%, umfasst.

8. Verfahren nach einem der vorstehenden Ansprüche, wobei die Katalysatorteilchen ferner eine Matrix und insgesamt 60 Gew.-% oder weniger der ersten und der zweiten Molekularsiebe, bezogen auf die gesamten Katalysatorteilchen, vorzugsweise im Bereich von 15 bis 50 Gew.-%, umfassen.

9. Verfahren nach einem der vorstehenden Ansprüche, wobei die Umsetzung des Oxygenat-Einsatzmaterials in Gegenwart eines Olefin-Co-Einsatzmaterials erfolgt.

## Revendications

1. Procédé de préparation d'un produit oléfinique, lequel procédé comprend la mise en réaction d'une charge de composé oxygéné dans une zone de réaction en présence de particules de catalyseur, les particules de catalyseur individuelles comprenant à la fois un premier tamis moléculaire possédant des canaux unidimensionnels formés par des noyaux à 10 chaînons et un second tamis moléculaire possédant des canaux multidimensionnels, pour préparer un produit de réaction oléfinique.

2. Procédé selon la revendication 1, dans lequel le premier tamis moléculaire a un rapport silice sur alumine de moins de 200, de préférence de 150 ou moins.

3. Procédé selon l'une quelconque des revendications précédentes, dans lequel une distance moyenne entre un cristal du premier tamis moléculaire et un cristal le plus proche du second tamis moléculaire est inférieure à une granulométrie moyenne des particules de catalyseur, de préférence de 40 µm ou moins, de préférence de 20 µm ou moins, en particulier de 10 µm ou moins.

4. Procédé selon l'une quelconque des revendications précédentes, dans lequel le tamis moléculaire ayant des canaux unidimensionnels formés par noyaux à 10 chaînons comprend au moins un parmi un tamis moléculaire de type MTT et un tamis moléculaire du type TON, en particulier de la zéolite ZSM-13 et/ou de la zéolite ZSM-22.

5. Procédé selon l'une quelconque des revendications précédentes, dans lequel le second tamis moléculaire est un tamis moléculaire de type MFI, en particulier de la zéolite ZSM-5.

6. Procédé selon la revendication 5, dans lequel le second tamis moléculaire a un rapport silice sur alumine (SAR) d'au moins 60, de préférence d'au moins 80, plus préférablement d'au moins 100, encore plus préférablement d'au moins 150.

7. Procédé selon l'une quelconque des revendications précédentes, dans lequel un lit de particules de catalyseur comprend au moins 1% en poids et au moins 50% en poids du second tamis moléculaire, rapportés au poids total des premier et second tamis moléculaires dans la composition de catalyseur ; de préférence au moins 5% en poids et moins de 40% en poids, plus préférablement au moins 8% en poids et moins de 25% en poids.

8. Procédé selon l'une quelconque des revendications précédentes, dans lequel les particules de catalyseur comprennent en outre une matrice et un total de 60% en poids ou moins des premier et second tamis moléculaires, rapporté aux particules de catalyseur totales, de préférence dans la gamme allant de 15 à 50% en poids.

9. Procédé selon l'une quelconque des revendications précédentes, dans lequel la réaction de la charge d'alimentation de composés oxygénés est réalisée en présence d'une cocharge oléfinique.
